(19) Europäisches Patentamt
European Patent Office
Office européen des brevets

(11) Veröffentlichungsnummer: **0 088 320**
**B1**

(12) **EUROPÄISCHE PATENTSCHRIFT**

(45) Veröffentlichungstag der Patentschrift: **04.01.89**

(51) Int. Cl.⁴: **C 07 D 501/46,** A 61 K 31/545 // C07D277/20, C07D501/34

(21) Anmeldenummer: **83101915.3**

(22) Anmeldetag: **26.02.83**

(54) **Cephalosporinderivate und Verfahren zu ihrer Herstellung.**

(30) Priorität: **04.03.82 DE 3207840**

(43) Veröffentlichungstag der Anmeldung: **14.09.83 Patentblatt 83/37**

(45) Bekanntmachung des Hinweises auf die Patenterteilung: **04.01.89 Patentblatt 89/1**

(84) Benannte Vertragsstaaten: **AT BE CH DE FR GB IT LI LU NL SE**

(56) Entgegenhaltungen:
**EP-A-0 034 760**
**EP-A-0 064 740**
**GB-A-2 025 398**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Lattrell, Rudolf, Dr., Heuhohlweg 6H, D-6240 Königstein/Taunus (DE)**
Erfinder: **Kirrstetter, Reiner, Dr., Am Flachsland 56, D-6233 Kelkheim (Taunus) (DE)**
Erfinder: **Schwab, Wilfried, Dr., Am Flachsland 18, D-6233 Kelkheim (Taunus) (DE)**
Erfinder: **Dürckheimer, Walter, Dr., Im Lerchenfeld 45, D-6234 Hattersheim am Main (DE)**
Erfinder: **Seibert, Gerhard, Dr., Gläserweg 21, D-6100 Darmstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 088 320 B1

LIBER, STOCKHOLM 1989

**Beschreibung**

Die Erfindung betrifft neue Cephalosporinderivate und Verfahren zu ihrer Herstellung, insbesondere polare Cephemderivate, die in 3-Stellung des Cephemrings durch bestimmte Pyridiniummethyl-Reste substituiert sind und die eine sehr gute antimikrobielle wirkung gegen gram-positive und gram-negative Bakterien besitzen und deshalb als Arzneimittel zur Behandlung von mikrobiellen Infektionen geeignet sind.

Gegenstand der Erfindung sind daher Cephemderivate der allgemeinen Formel I

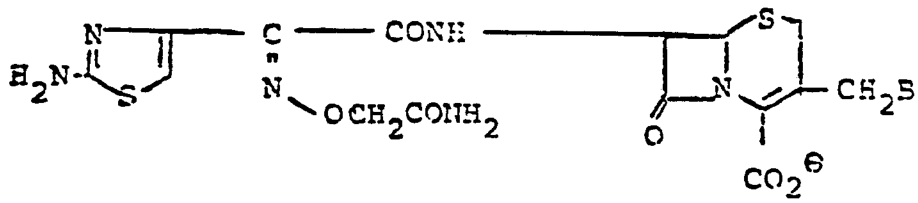

(I)

und deren Physiolosisch verträgliche Säureadditionssalze worin bedeuten

B einen Pyridiniumrest

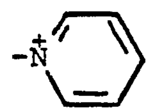

,

der ein- oder zweifach, gleich oder verschieden substituiert sein kann,
durch $C_1$-$C_4$-Alkyl, das einfach substituiert sein kann durch Hydroxy, und wobei 2 Alkylgruppen auch zu einem drei bis fünf Methylengruppen enthaltenden Ring geschlossen sein können,
durch $C_3$-$C_6$-Cycloalkyl oder durch Halogen und wobei die $H_2NCOCH_2O$-Gruppe in syn-Position steht.

Der an den Pyridiniumrest ankondensierte Ring kann 3 bis 5 Ringglieder enthalten und somit beispielsweise einen Cyclopenteno-, Cyclohexeno- oder Cyclohepteno-Ring darstellen.

Als besonders bevorzugt kommen für B beispielsweise die folgenden Bedeutungen in Betracht:
ein Pyridiniumrest, der 1- bis 2-fach substituiert sein kann durch $C_1$-$C_4$-Alkyl, wie insbesondere Methyl, Äthyl, Propyl, Isopropyl, n-Butyl, sec.-Butyl, tert.-Butyl, Dimethyl, Methyl und Äthyl, Methyl und Propyl, Methyl und Isopropyl, Äthyl und Äthyl,
durch Hydroxy-$C_1$-$C_4$-alkyl, wie insbesondere Hydroxymethyl, Hydroxyäthyl, Hydroxypropyl, Hydroyisopropyl, Hydroxybutyl, Hydroxy-sec.-butyl oder Hydroxy-tert.-butyl, durch $C_3$-$C_6$-Cycloalkyl, wie insbesondere Cyclopropyl, Cyclobutyl, Cyclopentyl oder Cyclohexyl, oder durch Halogen, wie insbesondere Fluor, Chlor, Brom oder Jod.

Gegenstand der Erfindung ist weiterhin ein Verfahren zur Herstellung von Verbindungen der Formel I und ihrer physiologisch verträglichen Säureadditionssalze, das dadurch gekennzeichnet ist, daß man

a) eine Verbindung der allgemeinen Formel II

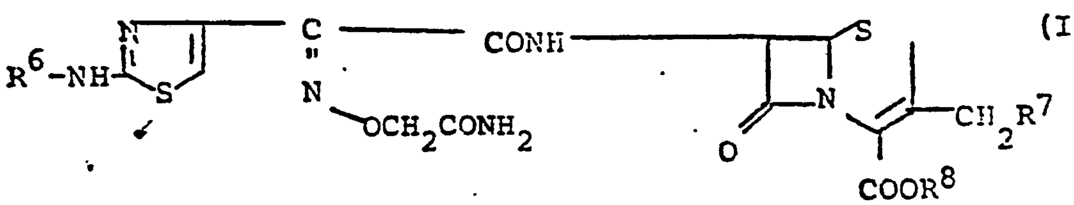

(II)

worin
$R^6$ Wasserstoff oder eine Aminoschutzgruppe
$R^7$ eine durch Pyridin oder substituierte Pyridine, die den Pyridiniumresten B der Formel I entsprechen, austauschbare Gruppe bedeutet,
$R^8$
ein Wasserstoffatom oder ein Äquivalent eines Alkalimetalls, Erdalkalimetalls, Ammonium oder einer organischen Aminbase bedeutet,
mit Pyridin oder einem solchen Pyridinderivat, das einem der in Formel I genannten Pyridiniumreste B entspricht, umsetzt, worauf, falls der Rest $R^6$ die obengenannte leicht abspaltbare Gruppe bedeutet, diese durch Behandlung mit einem Mittel zur sauren Hydrolyse, Hydrogenolyse oder mit Thioharnstoff abgespalten

und so eine Verbindung der Formel I erhalten wird, oder

b) eine 7-Amino-cephemverbindung der allgemeinen Formel III

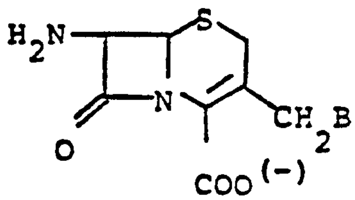

(III)

worin B die in Formel I genannte Bedeutung hat, oder deren Säureadditionssalze, wobei die Aminogruppe auch in Form eines reaktionsfähigen Derivats vorliegen kann, mit einer 2-(2-Aminothiazol-4-yl)-2-syn-oximino-essigsäure der allgemeinen Formel IV,

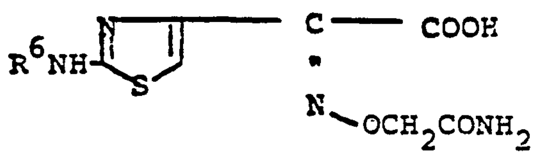

(IV)

worin
$R^6$ die oben genannte Bedeutung hat, oder mit einem aktivierten Derivat dieser Verbindung umsetzt und
α) eine gegebenenfalls vorhandene Schutzgruppe abspaltet und
β) falls erforderlich, das erhaltene Produkt in ein physiologisch verträgliches Säureadditionssalz überführt.

Soll die Herstellung der Verbindungen der allgemeinen Formel I durch nucleophilen Austausch von $R^7$ in den Verbindungen der allgemeinen Formel II durch Pyridin oder eines der angegebenen Pyridinderivate erfolgen, so kommen als Reste $R^7$ insbesondere in Betracht Acyloxyreste von niederen aliphatischen Carbonsäuren, vorzugsweise mit 1 bis 4 C-Atomen, wie z. B. Acetoxy oder Propionyloxy, insbesondere Acetoxy, die gegebenenfalls substituiert sein können, wie z. B. Chloracetoxy oder Acetylacetoxy. Für $R^7$ kommen auch andere Gruppen in Betracht, wie beispielsweise Halogen, insbesondere Chlor oder Brom, oder Carbamoyloxy.

Erfindungsgemäß werden bei der nucleophilen Austauschreaktion Ausgangsverbindungen der allgemeinen Formel II, in denen $R^7$ für Acetoxy steht, eingesetzt, oder deren Salze, wie z. B. ein Natrium- oder Kaliumsalz. Die Reaktion wird in einem Lösungsmittel, vorzugsweise in Wasser, oder in einem Gemisch aus Wasser und einem mit Wasser leicht mischbaren organischen Lösungsmittel, wie z. B. Aceton, Dioxan, Acetonitril, Dimethylformamid, Dimethylsulfoxid oder Äthanol durchgeführt. Die Reaktionstemperatur liegt im allgemeinen im Bereich von etwa 10 bis etwa 100°C, vorzugsweise zwischen 20 und 80°C. Die Pyridinkomponente wird in Mengen zugegeben, die zwischen etwa äquimolaren Mengen und einem bis zu etwa 5-fachen Überschuß liegt. Der Austausch des Restes $R^7$ wird durch Anwesenheit von Neutralsalzionen, vorzugsweise von Jodid- oder Thiocyanationen, im Reaktionsmedium erleichtert. Insbesondere werden etwa 10 bis etwa 30 Äquivalente Kaliumjodid, Natriumjodid, Kaliumthiocyanat oder Natriumthiocyanat zugegeben. Die Reaktion wird vorteilhaft in der Nähe des Neutralpunktes, vorzugsweise bei einem pH-Wert im Bereich von etwa 5 bis etwa 8 durchgeführt.

Für den Fall, daß $R^7$ für eine Carbamoylgruppe steht, wird die Austauschreaktion analog durchgeführt. Steht $R^7$ für Halogen, insbesondere Brom, so erfolgt der Austausch in literaturbekannter Weise.

Enthalten die Verbindungen der Formel II eine Aminoschutzgruppe in dem Rest $R^6$, so werden die Schutzgruppen nach dem nucleophilen Austausch in an sich bekannter Weise durch saure Hydrolyse, durch Hydrogenolyse oder mit Thioharnstoff abgespalten.

Als Aminoschutzgruppen $R^6$ eignen sich z. B. gegebenenfalls substituiertes Alkyl, wie beispieisweise tert.-Butyl, -tert.-Amyl, Benzyl, p-Methoxybenzyl, Trityl, Benzhydryl, vorzugsweise Trityl, Trialkylsilyl, wie beispielsweise Trimethylsilyl, gegebenenfalls substituiertes aliphatisches Acyl, wie z. B. Formyl, Chloracetyl, Bromacetyl, Trichloracetyl und Trifluoracetyl, vorzugsweise Chloracetyl oder gegebenenfalls substituiertes Alkoxycarbonyl wie beispielsweise Trichloräthoxycarbonyl, Benzyloxycarbonyl oder tert.-Butyloxycarbonyl, vorzugsweise tert.-Butyloxycarbonyl und Benzyloxycarbonyl, sowie 2-Tetrahydropyranyl.

Die Acylierung der Verbindungen der allgemeinen Formel III oder von deren Additionssalzen, beispielsweise mit Chlorwasserstoffsäure, Bromwasserstoffsäure, Salpetersäure, Phosphorsäure, oder einer organischen Säure, wie z. B. Methansulfonsäure oder p-Toluolsulfonsäure, kann mit Carbonsäuren der allgemeinen Formel IV oder mit einem reaktionsfähigen Derivat einer solchen Säure durchgeführt werden. In manchen Fällen ist es dabei von Vorteil, die 2-Aminogruppe in den Verbindungen der allgemeinen Formel IV vor der Umsetzung mit den vorstehend genannten Aminoschutzgruppen $R^6$ zu schützen.

Die Schutzgruppe kann nach der Acylierung in an sich bekannter Weise abgespalten werden, z. B. die Tritylgruppe mittels einer Carbonsäure, wie z. B. Essigsäure, Trifluoressigsäure oder Ameisensäure, oder die Chloracetylgruppe mittels Thioharnstoff.

Werden die Carbonsäuren der allgemeinen Formel IV sowie ihre an der Aminogruppe geschützten Derivate selbst als Acylierungsmittel eingesetzt, so wird zweckmäßig in Gegenwart eines Kondensationsmittels, beispielsweise eines Carbodiimids, wie beispielsweise N,N'-Dicyclohexylcarbodiimid, gearbeitet.

Die Aktivierung der Carbonsäure der allgemeinen Formel IV kann in besonders güstiger Weise durch Behandlung mit bestimmten Carbonsäureamiden und beispielsweise Phosgen, Phosphorpentachlorid, Tosylchlorid, Thionylchlorid oder Oxalylchlorid erfolgen, wie sie in der deutschen Patentschrift 2 804 040 beschrieben wird.

Als aktivierte Derivate der Carbonsäuren der allgemeinen Formel IV eignen sich insbesondere auch Halogenide, vorzugsweise Chloride, die in an sich bekannter Weise durch Behandlung mit Halogenierungsmitteln, wie z. B. Phosphorpentachlorid, Phosgen oder Thionylchlorid unter für die Cephalosporinchemie literaturbekannten, schonenden Reaktionsbedingungen erhalten werden.

Als aktivierte Derivate der Carbonsäuren der allgemeinen Formel IV eignen sich ferner die Anhydride und gemischten Anhydride, Azide und aktivierten Ester, vorzugsweise mit p-Nitrophenol, 2,4-Dinitrophenol, Methylencyanhydrin, N-Hydroxysuccinimid und N-Hydroxyphthalimid, insbesondere diejenigen mit 1-Hydroxybenzotriazol und 6-Chlor-1-hydroxybenzotriazol. Als gemischte Anhydride sind besonders geeignet solche mit niederen Alkansäuren, wie z. B. Essigsäure, und besonders bevorzugt solche mit substituierten Essigsäuren, wie z. B. Trichloressigsäure, Pivalinsäure oder Cyanessigsäure. Besonders geeignet sind aber auch die gemischten Anhydride mit Kohlensäurehalbestern, die man beispielsweise durch Umsetzung der Carbonsäuren der Formel IV, in denen die Aminogruppe geschützt ist, mit Chlorameisensäure-benzylester, p-nitrobenzylester, -iso-butylester, -ethylester oder -allylester gewinnt. Die aktivierten Derivate können als isolierte Substanzen, aber auch in situ umgesetzt werden.

Im allgemeinen erfolgt die Umsetzung der Cephemderivate der allgemeinen Formel III mit einer Carbonsäure der allgemeinen Formel IV oder einem aktivierten Derivat derselben in Gegenwart eines inerten Lösungsmittels. Insbesondere eignen sich chlorierte Kohlenwasserstoffe, wie vorzugsweise Methylenchlorid und Chloroform; Äther, wie z. B. Diäthyläther, vorzugsweise Tetrahydrofuran und Dioxan; Ketone, wie vorzugsweise Aceton und Butanon; Amide, wie vorzugsweise Dimethylformamid und Dimethylacetamid oder Wasser. Es kann sich auch als vorteilhaft erweisen, Gemisch der genannten Lösungsmittel zu verwenden. Dies ist oftmals dann der Fall, wenn die Cephemverbindung der allgemeinen Formel III mit einem in situ erzeugten aktivierten Derivat einer Carbonsäure der Formel IV umgesetzt wird.

Die Umsetzung von Cephemverbindungen der Formel III mit Carbonsäuren der Formel IV bzw. deren aktivierten Dervaten kann in einem Temperaturbereich von etwa -80 bis etwa +80°C vorzugsweise zwischen -30 und +50°C, insbesondere jedoch zwischen etwa -20°C und Raumtemperatur erfolgen.

Die Reaktionsdauer hängt von den Reaktanten, der Temperatur und dem Lösungsmittelgemisch ab und liegt normalerweise zwischen etwa 1/4 und etwa 72 Stunden.

Die Reaktion mit Säurehalogeniden kann gegebenenfalls in Gegenwart eines säurebindenden Mittels zur Bindung des freigesetzten Halogenwasserstoffs durchgeführt werden. Als solche eignen sich insbesondere tertiäre Amine, wie z. B. Triäthylamin oder Dimethylanilin, anorganische Basen, wie z. B. Kaliumcarbonat oder Natriumcarbonat, Alkylenoxide, wie z. B. Propylenoxid. Auch die Anwesenheit eines Katalysators, wie z. B. von Dimethylaminopyridin kann gegebenenfalls von Vorteil sein.

Liegt in den Verbindungen der allgemeinen Formel III die Aminogruppe in Form eines reaktionsfähigen Derivats vor, so kann es sich um ein solches handeln, wie es aus der Literatur für Amidierungen bekannt ist. So kommen beispielsweise Silylderivate in Betracht, die bei der Umsetzung von Verbindungen der allgemeinen Formel III mit einer Silylverbindung gebildet werden, wie z. B. Trimethylchlorsilan oder Bis-(trimethylsilyl)-acetamid. Wird die Umsetzung mit einer solchen, an der Aminogruppe aktivierten Verbindung durchgeführt, so ist es zweckmäßig, die Reaktion in einem inerten Lösungsmittel, wie z. B. Methylenchlorid, Tetrahydrofuran oder Dimethylformamid durchzuführen.

Als physiologisch verträglichesäureadditionsalze der Verbindungen der allgemeinen Formel I seien beisbielsweise erwähnt solche mit Chlorwasserstoffsäure, Bromwasserstoffsäure, Salpetersäure, Phosphorsäure oder organische Säuren, wie z. B. Methansulfonsäure oder o-Toluolsulfonsäure oder Maleinsäure.

Die Verbindungen der allgemeinen Formel III können in an sich bekannter Weise, beispielsweise aus der an der Aminogruppe geschützten 7-Aminocephalosporansäure auf dieselbe Weie erhalten werden, wie sie verstehend für den nucleophilen Austausch von $R^7$ beschrieben wurde.

Die Verbindungen der allgemeinen Formel IV sowie die den Pyridiniumresten B entsprechenden Pyridinderivate sind literaturbekannt oder nach literaturbekannten Verfahren herstellbar.

Die erfindungsgemäß erhaltenen Vorbindungen der allgemeinen Formel I und ihre physiologisch verträglichen Säureadditionssalze zeigen bemerkenswert gute antibakterielle Wirksamkeiten, sowohl gegen grampositive als auch gramnegative bakterielle Keime.

Auch gegen penicillinase- und cephalosporinase-bildende Bakterien sind die Verbindungen der Formel I unerwartet gut wirksam. Da sie zudem günstige toxikologische und pharmakologische Eigenschaften zeigen, stellen sie wertvolle Chemotherapeutika dar.

Die Erfindung betrifft somit auch Arzneipräparate zur Behandlung von mikrobiellen Infektionen, die durch

einen Gehalt an einer oder mehreren der erfindungsgemäßen Verbindungen charakterisiert sind.

Die erfindungsgemäßen Produkte können auch in Kombination mit anderen Wirkstoffen, beispielsweise aus der Reihe der Penicilline, Cephalosporine oder Aminoglykoside zur Anwendung kommen.

Die Verbindungen der allgemeinen Formel I und ihre physiologisch verträglichen Säureadditionssalze können oral, intramuskulär oder intravenös verabfolgt werden. Arzneipräparate, die eine oder mehrere Verbindungen der allgemeinen Formel I als Wirkstoff enthalten, können hergestellt werden, indem man die Verbindungen der Formel I mit einem oder mehreren pharmakologisch verträglichen Trägerstoffen oder Verdünnungsmitteln, wie z. B. Füllstoffen, Emulgatoren, Gleitstoffen, Geschmackskorrigentien, Farbstoffen oder Puffersubstanzen vermischt und in eine geeignete galenische Zubereitungsform bringt, wie beispielsweise Tabletten, Dragees, Kapseln, oder eine zur parenteralen Applikation geeignete Suspension oder Lösung.

Als Träger- oder Verdünnungsmittel seien beispielsweise erwähnt Traganth, Milchzucker, Talkum, Agar-Agar, Polyglykole, Äthanol und Wasser. Für die parenterale Applikation kommen vorzugsweise Suspensionen oder Lösungen in Wasser in Betracht. Es ist auch möglich, die Wirkstoffe als solche ohne Träger- oder Verdünnungsmittel in geeigneter Form, beispielsweise in Kapseln, zu applizieren. Geeignete Dosen der Verbindungen der allgemeinen Formel I oder ihrer physiologisch verträglichen Säureadditionssalze liegen bei etwa 0,1 bis 20 g/Tag, vorzugsweise bei 0,5 bis 4 g/Tag für einen Erwachsenen von etwa 60 kg Körpergewicht.

Es können Einzel- oder im allgemeinen Mehrfachdosen verabreicht werden, wobei die Einzeldosis den Wirkstoff in einer Menge von etwa 50 bis 1000 mg vorzugsweise von etwa 100 bis 500 mg enthalten kann.

Cephemverbindungen der Formel I, in der B für Pyridinium und der Rest $-CH_2COOR'$ (R' = H oder $C_{1-4}$ Alkyl) an der Oximinogruppe steht, sind aus der deutschen Offenlegungsschrift 3 037 101 bekannt, solche mit B = Pyridinium oder 3- oder 4-Carbamoylpyridinium und einem Rest

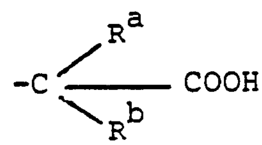

an der Oximinogruppe (mit $R_{a,b}$ = $C_{1-4}$-Alkyl oder $C_{3-7}$-Cycloalkyliden) aus der GB-A-2 025 398. Im Rest B und an der Oximinogruppe entsprechend substituierte Verbindungen, die jedoch am Aminothiazolring noch einen 5-Chlorsubstituenten tragen sind auf Seite 22 der EP-A-0 034 760 offenbart.

Erfindungsgemäß lassen sich außer den in den Ausführungsbeispielen beschriebenen Produkten beispielsweise auch Verbindungen herstellen, die der allgemeinen Formel I' entsprechen

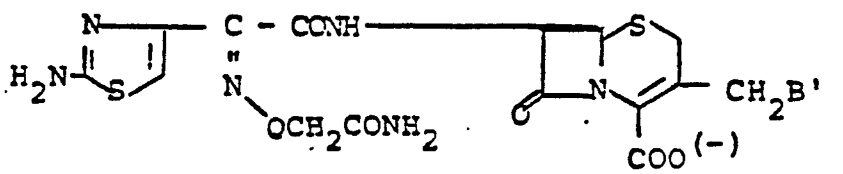 I'

in der der Rest $-CH_2CONH_2$ in syn-Position steht und B'

a) einen Pyridiniumrest darstellt, der durch die in Tabelle 1 angegebenen Reste substituiert ist oder
b) einen der in Tabelle 2 wiedergegebenen Reste bedeutet.

In Tabelle 1 geben die Zahlen die Stellung des (oder der) Substituenten am Pyridiniumrest wieder.

**Tabelle 1**

2,3-di-$CH_3$
2,5-di-$CH_3$
2-Propyl
3-Propyl
2-Isopropyl
2-n-Butyl
3-n-Butyl
4-n-Butyl
2-sec-Butyl
3-sec-Butyl
4-sec-Butyl

2-tert-Butyl
3-tert-Butyl
3-Cyclobutyl
4-Cyclobutyl
2-Cyclopentyl
3-Cyclopentyl
4-Cyclopentyl
2-Cyclohexyl
3-Cyclohexyl
4-Cyclohexyl
2-Cyclopentyl-3-$CH_3$
2-Cyclopentyl-4-$CH_3$
2-Cyclopentyl-5-$CH_3$
3-Cyclopentyl-4-$CH_3$
3-Cyclopentyl-5-$CH_3$
2-$CH_2OH$-3-$CH_3$
2-$CH_2OH$-4-$CH_3$
2-$CH_2OH$-5-$CH_3$
3-$CH_2OH$-2-$CH_3$
3-$CH_2OH$-4-$CH_3$
3-$CH_2OH$-5-$CH_3$
3-$CH_2OH$-6-$CH_3$
4-$CH_2OH$-2-$CH_3$
4-$CH_2OH$-3-$CH_3$
2-$CH_2OH$-3-$C_2H_5$
2-$CH_2OH$-4-$C_2H_5$
2-$CH_2OH$-5-$C_2H_5$
3-$CH_2OH$-2-$C_2H_5$
2-$C_2H_5$-3-$CH_3$
2-$C_2H_5$-4-$CH_3$
2-$C_2H_5$-5-$CH_3$
3-$C_2H_5$-2-$CH_3$
3-$C_2H_5$-5-$CH_3$
4-$C_2H_5$-2-$CH_3$
4-$C_2H_5$-3-$CH_3$
2-Cyclopropyl
3-Cyclopropyl
4-Cyclopropyl
2-Cyclobutyl
2-$CH_2CH_2OH$
3-$CH_2CH_2OH$
4-$CH_2CH_2OH$
2-$CH_2CH_2OH$-4-$CH_3$
2-$CH_2CH_2OH$-3-$CH_3$
2-$CH_2CH_2OH$-5-$CH_3$
3-$CH_2CH_2OH$-2-$CH_3$
3-$CH_2CH_2OH$-4-$CH_3$
3-$CH_2CH_2OH$-5-$CH_3$
3-$CH_2CH_2OH$-6-$CH_3$
4-$CH_2CH_2OH$-3-$CH_3$
4-$CH_2CH_2OH$-2-$CH_3$
2-$CH(CH_3)OH$-3-$CH_3$
2-$CH(CH_3)OH$-4-$CH_3$
2-$CH(CH_3)OH$-5-$CH_3$
3-$CH(CH_3)OH$-2-$CH_3$
3-$CH(CH_3)OH$-4-$CH_3$
3-$CH(CH_3)OH$-5-$CH_3$
3-$CH(CH_3)OH$-6-$CH_3$
4-$CH(CH_3)OH$-2-$CH_3$
4-$CH(CH_3)OH$-3-$CH_3$
2-$CH(C_2H_5)OH$
3-$CH(C_2H_5)OH$
4-$CH(C_2H_5)OH$
4-Cyclopentyl-2-$CH_3$
4-Cyclopentyl-3-$CH_3$

$2\text{-}CH_2CH(OH)CH_3\text{-}4\text{-}CH_3$
$2\text{-}C(CH_3)_2OH\text{-}4\text{-}CH_3$
$3\text{-}C(CH_3)_2OH\text{-}6\text{-}CH_3$
$4\text{-}C(CH_3)_2OH\text{-}3\text{-}CH_3$
$2\text{-}CH(CH_3)OH\text{-}4\text{-}C_2H_5$
$2\text{-}CH_2CH_2OH\text{-}5\text{-}C_2H_5$
$2\text{-}CH(C_3H_7)OH$
$3\text{-}CH(C_3H_7)OH$
$4\text{-}CH(C_3H_7)OH$
$2\text{-}CH(C_2H_5)CH_2OH$
$3\text{-}CH(C_2H_5)CH_2OH$
$4\text{-}CH(C_2H_5)CH_2OH$
$2\text{-}CH_2(CH_2)_3OH$
$3\text{-}CH_2(CH_2)_3OH$
$4\text{-}CH_2(CH_2)_3OH$
$2\text{-}CH(CH_3)CH_2CH_2OH$
$3\text{-}CH(CH_3)CH_2CH_2OH$
$4\text{-}CH(CH_3)CH_2CH_2OH$

$CH_3$
|
$2\text{-}C(C_2H_5)OH$

$CH_3$
|
$3\text{-}C(C_2H_5)OH$

$CH_3$
|
$4\text{-}C(C_2H_5)OH$

$2\text{-}CH_2C(CH_3)_2OH$
$3\text{-}CH_2C(CH_3)_2OH$
$4\text{-}CH_2C(CH_3)_2OH$
$2\text{-}CH_2OH\text{-}3\text{-}Br$
$4\text{-}CH_2OH\text{-}3\text{-}Br$
$5\text{-}CH_2OH\text{-}3\text{-}Br$
$3\text{-}CH_2OH\text{-}4\text{-}C_2H_5$
$3\text{-}CH_2OH\text{-}5\text{-}C_2H_5$
$3\text{-}CH_2OH\text{-}6\text{-}C_2H_5$
$4\text{-}CH_2OH\text{-}2\text{-}C_2H_5$
$4\text{-}CH_2OH\text{-}3\text{-}C_2H_5$
2,3-di-$CH_2OH$
2,5-di-$CH_2OH$
2,4-di-$CH_2OH$
3,4-di-$CH_2OH$
3,5-di-$CH_2OH$
$2\text{-}CH(CH_3)CH_2OH$
$3\text{-}CH(CH_3)CH_2OH$
$4\text{-}CH(CH_3)CH_2OH$
$4\text{-}CH(CH_3)CH_2OH\text{-}5\text{-}CH_3$
$2\text{-}(CH_2)_3OH$
$3\text{-}(CH_2)_3OH$
$4\text{-}(CH_2)_3OH$
$2\text{-}CH_2CH(OH)CH_3$
$3\text{-}CH_2CH(OH)CH_3$
$4\text{-}CH_2CH(OH)CH_3$
$6\text{-}CH_2OH\text{-}3\text{-}Br$
$2\text{-}CH_2OH\text{-}3\text{-}Cl$
$4\text{-}CH_2OH\text{-}3\text{-}Cl$
$5\text{-}CH_2OH\text{-}3\text{-}Cl$
$6\text{-}CH_2OH\text{-}3\text{-}Cl$
$2\text{-}CH_2OH\text{-}3\text{-}F$
$5\text{-}CH_2OH\text{-}3\text{-}F$
$4\text{-}CH_2OH\text{-}3\text{-}F$
$6\text{-}CH_2OH\text{-}3\text{-}F$

**Tabelle 2**

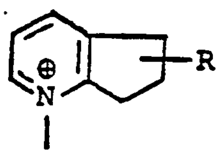

R=H

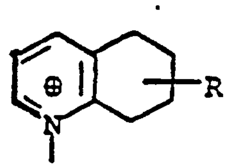

R=H

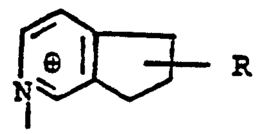

R=H

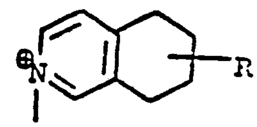

R=H

Die folgenden Ausführungsbeispiele für erfindungsgemäß herstellbare syn-Verbindungen dienen zur weiteren Erläuterung der Erfindung.

**Referenzbeispiel**

a) <u>(6R,7R)-3-Acetoxymethyl-7-[(Z)-2-(2-aminothiazol-4-yl)-2-carboxymethyloxyimino-acetomido]-ceph-3-em-4-corbonsäure, trifluorocetat</u>
Eine Lösung von 1,7 g (2 mmol) (6R,7R)-3-Acetoxymethyl-7-[(Z)-2-tert.-butoxycarbonylmethyloxyimino-2-(2-tritylaminothiazol-4-yl)acetamido]-ceph-3-em-4-corbonsäure-tert.-butylester in 20 ml Trifluoressigsäure wird 30 Minuten bei Raumtemperatur gerührt. Es wird eingeengt, der Rückstand mit Äther/n-Penton (2 : 1) digeriert, abgesaugt mit demselben Lösungsmittelgemisch gewaschen und an der Luft getrocknet. Dit Ausbeute ist quantitativ.

b) <u>(6R,7R)-7-[(Z)-2-(2-Aminothiazol-4-yl)-2-(carboxymethyloxyimino)-acetamido]-3-[(2,3-cyclopenteno-1-pyridinium) methyl]-ceph-3-em-4-carboxylat-Monokaliumsalz</u>

1,2 g (2 mmol) Produkt der Stufe a) werden in 4 ml Wasser suspendiert und durch Zugabe von 0,6 g Kaliumhydrogencarbonat in Lösung gebracht. Sodann werden 3,9 g (40 mmol) Kaliumrhodanid und 0,71 ml (6 mmol) 2,3-Cyclopentenopyridin zugegeben und der pH der Mischung mit 85 proz. Phosphorsäure auf 6,6 eingestellt. Es wird 3 Stunden auf 65 - 70° erhitzt. Die Mischung wird mit 32 ml Aceton verdünnt, von wenig Ungelöstem wird filtriert und das Filtrat über 200 g Kieselgel (Merck 0,063 - 0,2 mm) chromatographiert. Mit Aceton/Wasser (8 : 1) werden die Neutralsalze, mit Aceton/Wasser (2 : 1) das produkt: eluiert. Das gefriergetracknete Rohpradukt wird an Kieselgel (Merck, Lobar B-Säule, Art 10401, ca. 1 bar, Aceton/Wasser 2: 1) rechromatographiert. Durch Gefriertrocknen der Produktfraktionen (Fr. 7 - 10, 60 ml) erhält man 0,25 g. (21 % d.Th.) der Titelverbindung in Form eines farblasen Feststoffs.

Nach dem Verfahren des Referenzbeispiels werden die nachfolgend aufgeführten Verbindungen erhalten, die der allgemeinen Formel I entsprechen und im Pyridiniumrest (B in Formel I) die in der dritten Spalte der Tabelle 3 angegebenen Substituenten tragen. Die Zahl der Substituenten gibt jeweils die genaue Stellung des oder der Substituenten im Pyridiniumrest an.

**Tabelle 3**

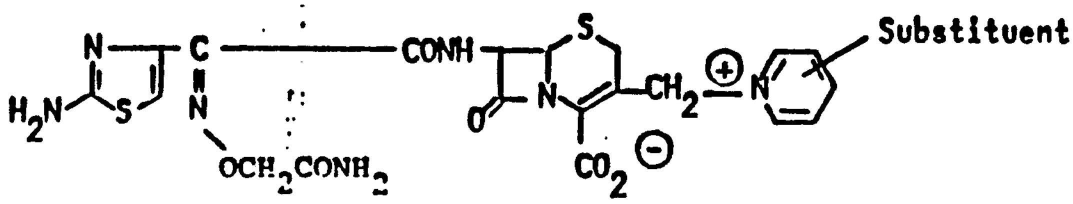

| Beispiel | Substituent | Verfahren Ausb. % d.Th. | $^1$H-NMR: δ (ppm) in $CF_3CO_2D$ |
|---|---|---|---|
| 1 | 2,3-Trimethylen | a (31) | 2,18-2,81 (m, 2H, Cyclapenten-H); 3,05-3,95 (m, 6H, 4 Cyclopenten-H und $SCH_2$); 4,92-6,25 (m, 6H, O-$CH_2$, $CH_2$Py und 2 Lactam-H); 7,46 (s, 1H, Thiazol); 7,66-8,75 (m, 3H,Py) |
| 2 | 4-Cyclopropyl | a (24) | 1,0-2,5 (m, 5H, Cyclopropyl-H); 3,43 und 3,79 (AB, J = 19 Hz, 2H, $SCH_2$); 5,06 (s, 2H, $OCH_2$); 5,15-6,30 (m, 4H, $CH_2$Py und 2 Lactam-H); 7,43 (s, 1H, Thiazol); 7,63 und 8,73 (AA', BB', J = 4H, Py) |
| 3 | H | a (31) | 3,55 und 3,83 (AB, J = 19 Hz, 2H, $SCH_2$); 5,06 (s, 2H, $OCH_2$); 5,16-6,25 (m, 4H, $CH_2$Py und 2 Lactam-H); 7,41 (s, 1H, Thiazal); 7,7-9,2 (m, 5H, Py) |
| 4 | 3-$CH_3$ | a (28) | 2,68 (s, 3H, $CH_3$); 3,48 und 3,78 (AB, J = 19 Hz, 2H, $SCH_2$); 5,07 (s, 2H, $OCH_2$); 5,16-6,35 (m, 4H, $CH_2$Py und 2 Lactam-H); 7,43 (s, 1H, Thiazol); 7,72-9,00 (m, 4H, Py) |
| 5 | 3-$CH_2OH$ | a (34) | 3,52 und 3,82 (AB, J = 19 Hz, 2H, $SCH_2$); 5,06 (s, 2H, $OCH_2$); 5,20 (s, 2H, $CH_2OH$); 5,1-6,4 (m, 4H, $CH_2$Py und 2 Lactam-H); 7,43 (s, 1H, Thiazol) 7,72-9,25 (m, 4H, Py) |
| 6 | 3-Cl | a( 25) | 3,55 und 3,88 (AB, J = 19 Hz, 2H, $SCH_2$); 5,08 (s, 2H, $OCH_2$); 5,2-6,4 (m, 4H, $CH_2$Py und 2 Lactam-H); 7,44 (s, 1H, Thiazol); 7,7-9,25 (m, 4H, Py). |

**Patentansprüche** für die Vertragsstaaten: BE, CH, DE, FR, GB, IT, LI, LU, NL, SE

1. Cephemderivate der allgemeinen Formel I

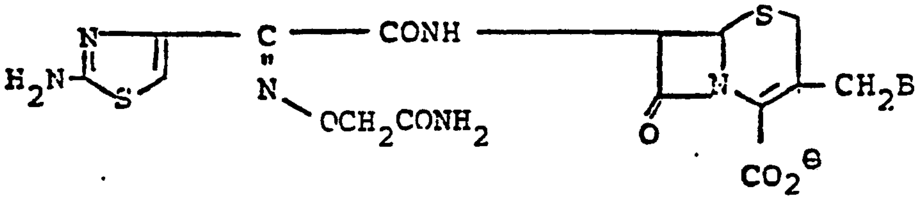 (I)

und deren physiologisch verträgliche Säureadditionssalze worin bedeuten

B einen Pyridiniumrest-

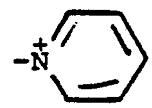 ,

der ein- oder zweifach, gleich oder verschieden substituiert sein kann,
durch $C_1$-$C_4$-Alkyl, das einfach substituiert sein kann durch Hydroxy, und wobei 2 Alkylgruppen auch zu einem drei bis fünf Methylengruppen enthaltenden Ring geschlossen sein können,
durch $C_3$-$C_6$-Cycloalkyl oder
durch Halogen,
und in der die $H_2NCOCH_2O$-Gruppe in syn-Position steht.

2. Verfahren zur Herstellung von Cephemverbindungen der Formel I und ihrer physiologisch verträglichen Säureadditionssalze, dadurch gekennzeichnet, daß man

a) eine Verbindung der allgemeinen Formel II

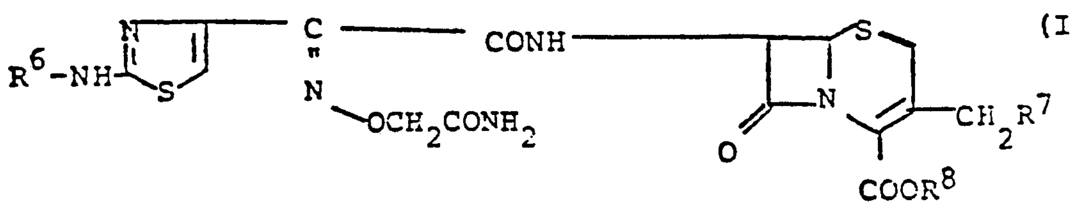 (II) .

worin
$R^6$ Wasserstoff oder eine Aminoschutzgruppe
$R^7$ eine durch Pyridin oder substituiertes Pyridine, die den Pyridiniumresten B der Formel I entsprechen, austauschbare Gruppe bedeutet,
$R^8$ ein Wasserstoffatom oder ein Äquivalent eines Alkalimetalls, Erdalkalimetalls, Ammonium oder einer organischen aminbase bedeutet,

mit Pyridin oder einem solchen Pyridinderivat, das einem der in Formel I genannten Pyridiniumreste B entspricht, umsetzt, worauf, falls der Rest $R^6$ die obengenannte leicht abspaltbare Gruppe bedeutet, diese durch Behandlung mit einem Mittel zur sauren Hydrolyse, Hydrogenolyse oder mit Thioharnstoff abgespalten und so eine Verbindung der Formel I erhalten wird, oder

b) eine 7-Amino-cephemverbindung der allgemeinen Formel III

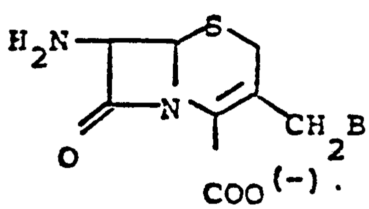 (III)

worin B die in Formel I genannte Bedeutung hat, oder deren Säureadditionssalze, wobei die Aminogruppe auch in Form eines reaktionsfähigen Derivats vorliegen kann, mit einer 2-(2-Aminothiazol-4-yl)-2-syn-oximino-essigsäure der allgemeinen Formel IV,

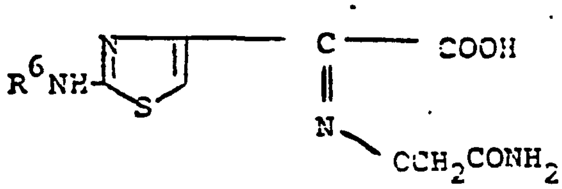 (IV)

worin
$R^6$ die obengenannte Bedeutung hat, oder mit einem aktivierten Derivat dieser Verbindung umsetzt und
α) eine gegebenenfalls vorhandene Schutzgruppe abspaltet und
β) falls erforderiich, das erhaltene Produkt in ein physiologisch verträgliches Säureadditionssalz überführt.

3. Verfahren gemäß Anspruch 2, dadurch gekennzeichnet, daß der nucleophile Austausch des Substituenten $R^7$ in Gegenwart von Neutraisalzionen, insbesondere von Jodid oder Thiocyanationen erfolgt.

4. Gegen bakterielle Infektionen wirksame pharmazeutische Präparate, gekennzeichnet durch einen Gehalt an Cephemderivaten der allgemeinen Formel I.

5. Verfahren zur Herstellung von gegen bakterielie Infektionen wirksamen pharmazeutischen Präparaten, dadurch gekennzeichnet, daß ein Cephemderivat der allgemeinen Formel I gegebenenfalls mit pharmazeutisch üblichen Trägerstoffen oder Verdünnungsmitteln in eine pharmazeutisch geeignete Verabreichungsform gebracht wird.

6. Verwendung von Cephemderivaten der allgemeinen Formel I zur Bekämpfung bakterieller Infektionen.

**Patentansprüche** für den Vertragsstaat: AT

1. Verfahren zur Herstellung von Cephemderivaten der allgemeinen Formel I

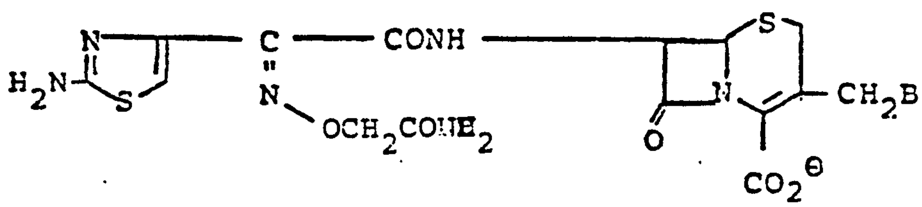

(I)

und deren physiologisch verträgliche Säureadditionssalze worin bedeuten

B einen Pyridiniumrest

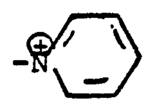

,

der ein- oder zweifach, gleich oder verschieden substituiert sein kann,
durch $C_1$-$C_4$-Alkyl, das einfach substituiert sein kann durch Hydroxy, wobei 2 Alkylgruppen auch zu einem drei bis fünf Methylengruppen enthaltenden Ring geschlossen sein können,
durch $C_3$-$C_6$-Cycloalkyl oder
durch Halogen,
und in der die Gruppe $-OCH_2CONH_2$ in syn-Position steht, dadurch gekennzeichnet, daß man

a) eine Verbindung der allgemeinen Formel II

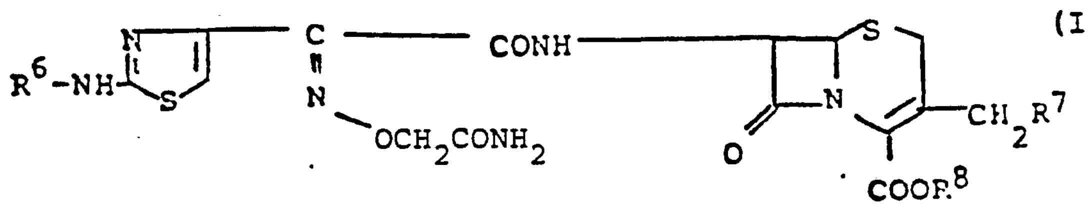

(II)

worin
$R^6$ Wasserstoff oder eine Aminoschutzgruppe
$R^7$ eine durch Pyridin oder substituiertes Pyridine, die den Pyridiniumresten B der Formel I entsprechen, austauschbare Gruppe bedeutet,
$R^8$ ein Wasserstoffatom oder ein Äquivalent eines Alkalimetalls, Erdalkalimetalls, Ammonium oder einer organischen Aminbase bedeutet,
mit Pyridin oder einem solchen Pyridinderivat, das einem der in Formel I genannten Pyridiniumreste B entspricht, umsetzt, worauf, falls der Rest $R^6$ die obengenannte leicht abspaltbare Gruppe bedeutet, diese durch Behandlung mit einem Mittel zur sauren Hydrolyse, Hydrogenolyse oder mit Thioharnstoff abgespalten und so eine Verbindung der Formel I erhalten wird, oder

b) eine 7-Amino-cephemverbindung der allgemeinen Formel III

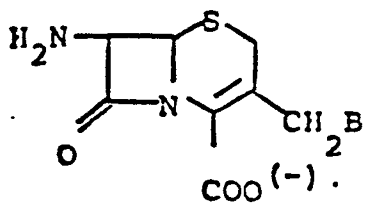

(III)

worin B die in Formel I genannte Bedeutung hat, oder deren Säureadditionssalze, wobei die Aminogruppe auch in Form eines reaktionsfähigen Derivats vorliegen kann, mit einer 2-(2-Aminothiazol-4-yl)-2-syn-oximino-essigsäure der allgemeinen Formel IV,

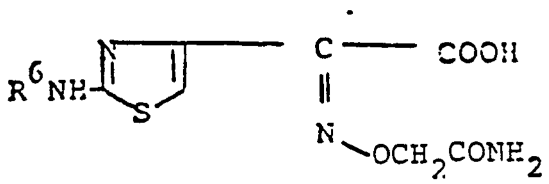

(IV)

worin

$R^6$ die obengenannte Bedeutung hat, oder mit einem aktivierten Derivat dieser Verbindung umsetzt und

α) eine gegebenenfalls vorhandene Schutzgruppe abspaltet und

β) falls erforderlich, das erhaltene Produkt in ein physiologisch verträgliches Säureadditionssalz überführt.

2. Verfahren gemäß Anspruch 1, dadurch gekennzeichnet, daß der nucleophile Austausch des Substituenten $R^7$ in Gegenwart von Neutralsalzionen, insbesondere von Jodid oder Thiocyanationen erfolgt.

**Claims** for the contracting ststes: BE, CH, DE, FR, GB, IT, LI, LU, NL, SI

1. A cephem derivative of the general formula I

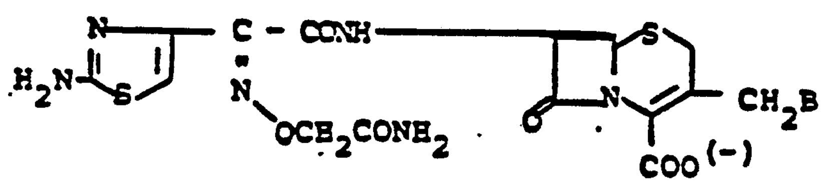

(I)

and its physiologically acceptable acid addition salts in which B denotes a pyridinium radical

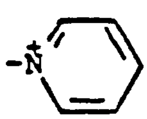

which can be monosubstituted or disubstituted by identical or different substituents, namely by $C_1$-$C_4$-alkyl, which can be monosubstituted by hydroxyl and of which 2 alkyl groups can also be linked to form a ring containing three to five methylene groups, by $C_3$-$C_6$-cycloalkyl or by halogen, and in which formula the $H_2NCOCH_2O$ group is in the syn position.

2. A process for preparing a cephem compound of the formula I and its physiologically acceptable acid addition salts, which comprises

a) reacting a compound of the general formula

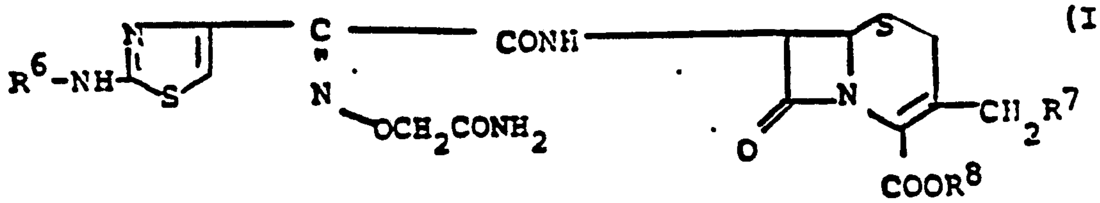

(II)

in which

$R^6$ denotes hydrogen or an amino-protecting group,

$R^7$ denotes a group which can be replaced by pyridine or substituted pyridines which correspond to the pyridinium radicals B of the formula I,

$R^8$ denotes a hydrogen atom or one equivalent of an alkali metal, alkaline earth metal, ammonium or of an organic amine base, with pyridine or a pyridine derivative which is such that it corresponds to one of the pyridinium radicals B mentioned in the formula I, whereupon, if the radical $R^6$ denotes the abovementioned readily detachable group, the latter is eliminated by treatment with an agent for acidic hydrolysis or hydrogenolysis or with thiourea and a compound of the formula I is thus obtained, or

b) reacting a 7-aminocephem compound of the general formula III

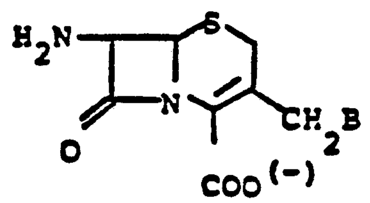

(III)

in which B has the meaning mentioned in the formula I, or its acid addition salts and in which the amino group can also be present in the form of a reactive derivative, with a 2-(2-aminothiazol-4-yl)-2-syn-oximinoacetic acid of the general formula IV

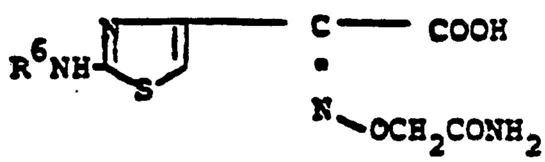

(IV)

in which

$R^6$ has the abovimentioned meaning, or with an activated derivative of this compound and

α) eliminating a protective group, if present, and

β) if necessary, converting the product obtained into a physiologically acceptable acid addition salt.

3. The process as claimed in claim 2, wherein the nucleophilic replacement of the substituent $R^7$ is effected in the presence of neutral salt ions, in particular iodide or thiocyanate ions.

4. A pharmaceutical formulation which is active against bacterial infections, containing a cephem derivative of the general formula I.

5. A process for preparing a pharmaceutical formulation which is active against bacterial infections, which comprises bringing a cephem derivative of the general formula I, if desired with pharmaceutically customary excipients or diluents, into a pharmaceutically suitable administration form.

6. The use of a cephem derivative of the general formula I, for combating bacterial infections.

**Claims** for the contracting state: AT

1. A process for preparing a cephem derivative of the general formula I

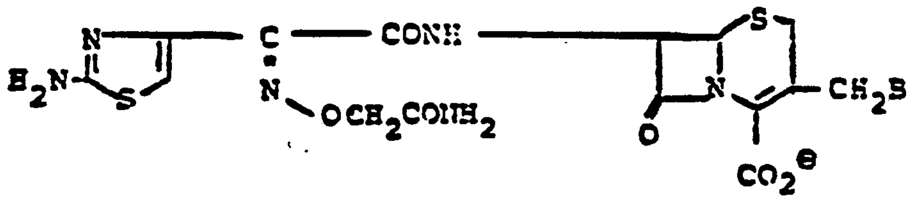

(I)

and its physiologically acceptable acid addition salts in which B denotes a pyridinium radical

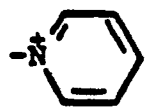

which can be monosubstituted or disubstituted by identical or different substituents, by $C_1$-$C_4$-alkyl, which can be monosubstituted by hydroxyl and of which 2 alkyl groups can also be linked to form a ring containing three to five methylene groups, by $C_5$-$C_6$-cycloalkyl or by halogen, and in which the group $-OCH_2CONH_2$ is in the syn position, which comprises

a) reacting a compound of the general formula II

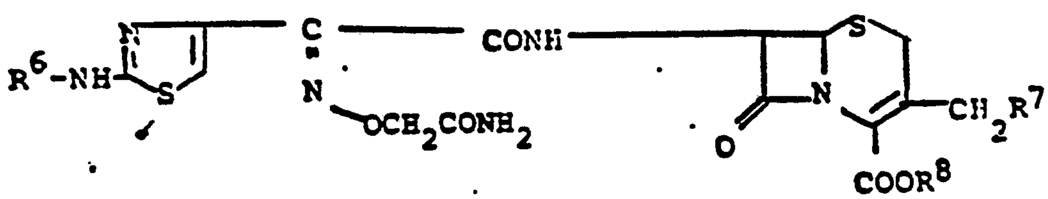

(II)

in which
$R^6$ denotes hydrogen or an amino-protecting group,
$R^7$ denotes a group which can be replaced by pyridine or substituted pyridines which correspond to the pyridinium radicals B of the formula I,
$R^8$ denotes a hydrogen atom or one equivalent of an alkali metal, alkaline earth metal, ammonium or of an organic amine base, with pyridine or a pyridine derivative which is such that it corresponds to one of the pyridinium radicals B mentioned in the formula I, whereupon, if the radical $R^6$ denotes the abovementioned readily detachable group, the latter is eliminated by treatment with an agent for acidic hydrolysis or hydrogenolysis or with thiourea and a compound of the formula I is thus obtained, or

b) reacting a 7-aminocephem compound of the general formula III

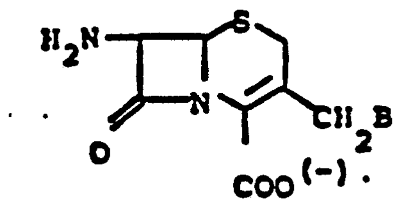

(III)

in which B has the meaning mentioned in the formula I, or its acid addition salts and in which the amino group can also be present in the form of a reactive derivative, with a 2-(2-aminothiazol-4-yl)-2-syn-oximinoacetic acid of the general formula IV

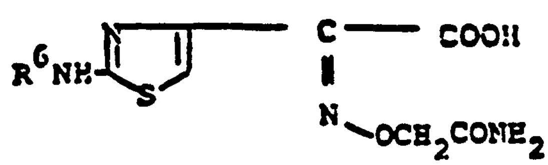

(IV)

in which
$R^6$ has the abovementioned meaning, or with an activated derivative of this compound and
α) eliminating a protective group, if present, and

β) if necessary, converting the product obtained into a physiologically acceptable acid addition salt.

2. The process as claimed in claim 1, wherein the nucleophilic replacement of the substituent $R^7$ is effected in the presence of neutral salt ions, in particular iodide or thiocyanate ions.

**Revendications** pour les Etats Contractants: BE, CH, DE, FR, GB, IT, LI, LU, NL, SE

1. Dérivés du céphéme répondant à la formule générale I

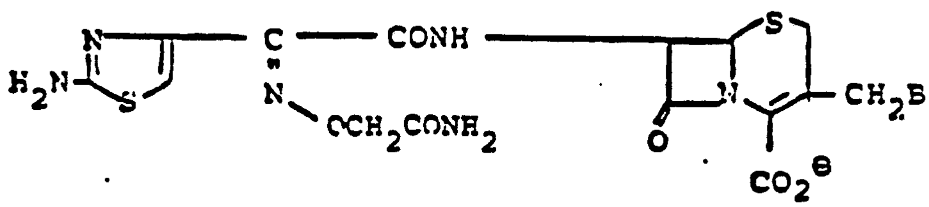

(I)

et leurs sels d'addition avec des acides physiologiquement acceptables, dans laquelle

B désigne un radical pyridinium

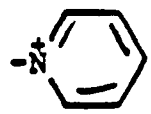

qui peut être substitué une ou deux fois de manière identique ou différente, par un groupe alkyle en $C_1$-$C_4$, qui peut être monosubstitué par un groupe hydroxy, deux groupes alkyle pouvant aussi se refermer pour donner un cycle contenant jusqu'à 3 à 5 groupes méthylène, par un groupe cycloalkyle en $C_3$-$C_6$ ou par un atome d'halogène,

et dans laquelle le groupe $H_2NCOCH_2O$-est en position syn.

2. Procédé de préparation descomposés du céphème répondant à la formule I et de leurs sels d'addition avec des acides physiologiquememt acceptables, caractérisé en ce que

a) on fait réagir un composé répondant à la formule générale II

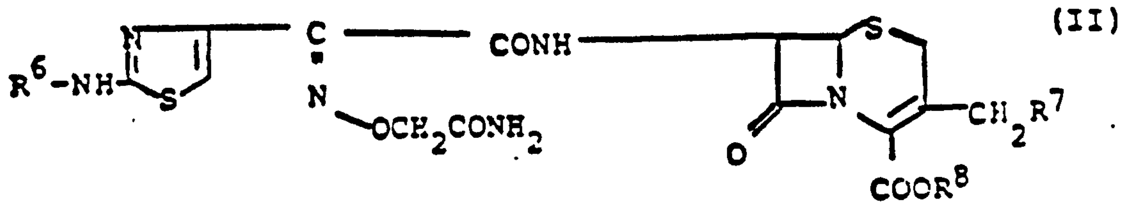

(II)

dans laquelle

$R^6$ désigne un atome d'hydrogène ou un groupe protecteur du groupe amino,

$R^7$ désigne un groupe échangeable par la pyridine ou par des pyridines substituees, qui correspondent aux radicaux pyridinium B de la formule I,

$R^8$ désigne un atome d'hydrogène ou un équivalent d'un métal alcalin, d'un métal alcalino-terreux, d'ammonium ou d'une base aminée organique,

avec de la pyridine ou un dérivé de la pyridine qui correspond à un des radicaux pyridinium B indiqués dans la formule I, apres quoi, si le radical $R^6$ désigne le groupe aisément éliminable indiqué ci-dessus, on l'élimine par traitement avec un agent pour l'hydrolyse acide, l'hydrogémolyse ou avec thiourée, et on obtient ainsi un composé répondant à la formule I, ou

b) on fait réagir un dérivé de 7-amino-céphème répondant à la formule générale III

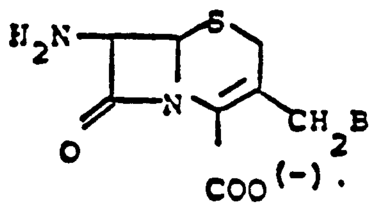

(III)

dans laquelle B a la signification indiquée dans la formule I, ou ses sels d'addition avec des acides, le groupe amino pouvant également être présent sous la forme d'un dérivé réactif, avec un acide 2-(2-aminothiazol-4-yl)-2-syn-oximino-acétique repondant à la formule générale IV,

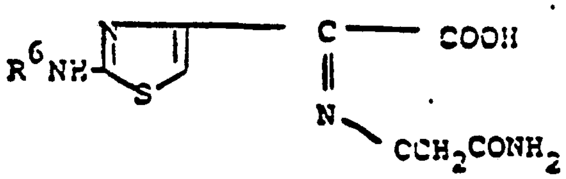

(IV)

dans laquelle

$R^6$ a la signification indiquée ci-dessus, ou avec un dérivé activé de ce composé, et

α) on élimine un groupe protecteur éventuellement présent et

β) si nécessaire, on transforme le produit obtenu en un sel d'addition avec un acide physiologiquement acceptable.

3. Procédé suivant la revendication 2, caractérisé en ce que l'échange nucléophile des substituants $R^7$ est effectué en présence d'ions de sels neutres, en particulier d'ions iodure ou thiocyanate.

4. Préparations pharmaceutiques actives contre les affections bactériennes, caractérisées en ce qu'elles contiennent des dérivés du céphème répondant à la formule générale I.

5. Procédé pour préparer des préparations pharmaceutiques actives contre les infections bactériennes, caractérisé en ce qu'on amène un dérivé du céphème répondant à la formule générale I, le cas échéant avec des véhicules ou diluants habituels en pharmacie, sous une forme d'administration pharmaceutiquement appropriée.

6. Utilisation de dérivés du céphème répondant à la formule générale I pour la lutte contre les infections bactériennes.

**Revendications** pour l'Etat Contractant: AT

1. Procédé de préparation de dérivés du céphème répondant à la formule générale I

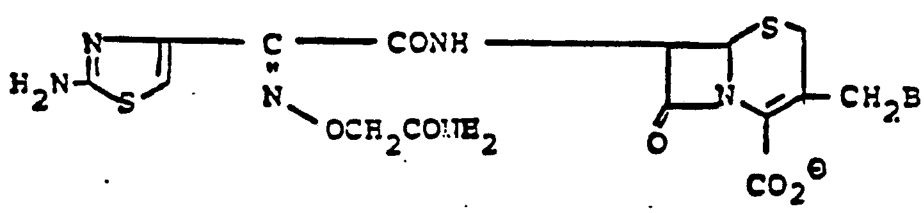

(I)

et de leurs sels d'addition avec des acides physiologiquement acceptables, dans laquelle

B désigne un radical pyridinium

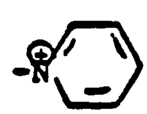

qui peut être substitué une ou deux fois de manière identique ou différente,

par un groupe alkyle en $C_1$-$C_4$, qui peut être monosubstitué par un groupe hydroxy, deux groupes alkyle pouvant également se refermer pour former un cycle contenant 3 à 5 groupes méthylène, par un groupe cycloalkyle en $C_3$-$C_6$ ou par un atome d'halogène,

et dans lequel le groupe $OCH_2CONH_2$ est en position syn, caractérisé en ce que

a) on fait réagir un composé répondant à la formule générale II

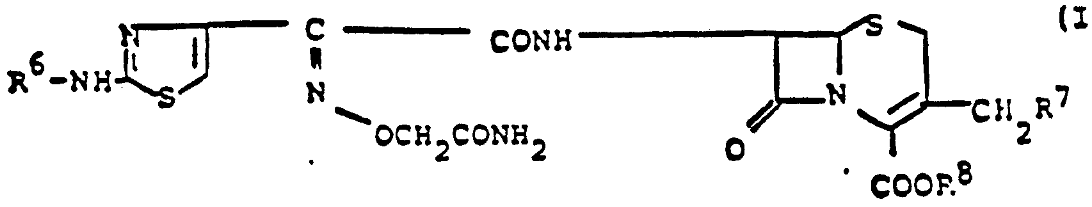

(II)

dans laquelle

$R^6$ désigne un atome d'hydrogène ou un groupe protecteur du groupe amino

$R^7$ désigne un groupe échangeable par une pyridine ou une pyridine substituée, qui correspond aux radicaux pyridinium B de la formule I,

$R^8$ désigne un atome d'hydrogène ou un équivalent d'un métal alcalin, d'un métal alcalino-terreux, d'ammonium ou d'une base aminée organique,

avec la pyridine ou un dérivé de la pyridine qui correspond à un des radicaux pyridinium B indiqués dans la formule I, après quoi, si le radical $R^6$ désigne le groupe aisément éliminable indiqué ci-dessus, on l'élimine par traitement avec un agent pour l'hydrolyse acide, l'hydrogénolyse ou avec de la thiourée et on obtient ainsi un composé répondant à la formule générale I, ou

b) on fait réagir un dérivé de 7-amino-céphème répondant à la formule générale III

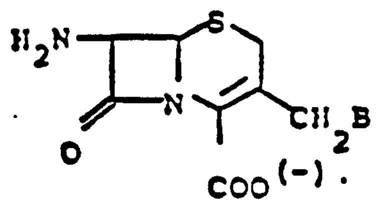

(III)

dans laquelle B a la signification indiquée dans la formule I, ou ses sels d'addition avec des acides le groupe amino pouvant également être présent sous la forme d'un dérivé réactif, avec un acide 2-(2-aminothiazol-4-yl)-2-syn-oximino-acétique répondant à la formule générale IV,

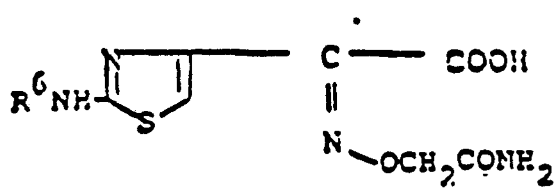

(IV)

dans laquelle

$R^6$ a la signification indiquée ci-dessus, ou avec un dérivé actif de ce composé, et

α) on élimine un groupe protecteur éventuellement présent et

β) si nécessaire, on transforme le produit obtenu en un sel d'addition avec un acide physiologiquement acceptables.

2. Procédé suivant la revendication 1, caractérisé en ce que l'échange nucléophile du substituant $R^7$ s'effectue en présence d'ions de sels neutres, en particulier d'ions iodure ou thiocyanate.